# EUROPEAN PATENT SPECIFICATION

(11) **EP 1 156 856 B1**
(45) Date of publication and mention of the grant of the patent: **31.08.2005**
(21) Application number: 00912515.4
(22) Date of filing: 28.02.2000
(51) Int. Cl.: A61P 25/00, A61K 31/4192

(54) **USE OF FLUORINATED TRIAZOLES FOR THE TREATMENT OF NEUROPATHIC PAIN**
VERWENDUNG VON FLUORIERTEN TRIAZOLEN ZUR BEHANDLUNG VON NEUROPATHISCHEM SCHMERZ
UTILISATION DE TRIAZOLES FLUORES DANS LE TRAITEMENT DES DOULEURS NEUROPATHIQUES

(30) Priority: 01.03.1999 US 259911; 01.03.1999 US 259910
(43) Date of publication of application: 28.11.2001
(73) Proprietor: Novartis AG, 4056 Basel (CH); Novartis Pharma GmbH, 1230 Wien (AT)
(72) Inventor: SCHMUTZ, Markus, CH-4124 Schönenbuch (CH)
(74) Representative: de Weerd, Petrus G.W.
(86) International application number: PCT/EP2000/001626
(87) International publication number: WO 2000/051577

(56) References cited:
- EP-A- 0 199 262
- DATABASE PHARMAPROJECTS [Online] "Rufinamide" Database accession no. 2514 XP002152066
- DATABASE PROMT [Online] "Novartis highlights clinical projects to turn around fortunes at its pharma division" Database accession no. 1999:641482 XP002152067 & MARKETLETTER, 4.10.99,

## Description

The present invention relates to a new pharmaceutical use of fluorinated triazoles.

More particularly the present invention relates to a new pharmaceutical use for compounds of formula I 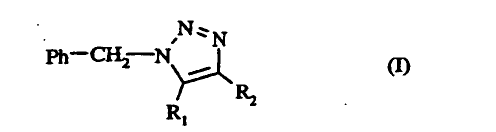 wherein Ph is an o-fluorinated phenyl radical which may be additionally substituted by 1 or 2 halogen atoms selected from fluorine and chlorine, R₁ is hydrogen, carbamoyl, N-(C₂-C₅)alkanoylcarbamoyl or N,N-di(C₁-C₄)alkylcarbamoyl, and R₂ is carbamoyl, N-(C₂-C₅)alkanoylcarbamoyl or N,N-di(C₁-C₄)alkylcarbamoyl.

The compounds of formula I as well as their production process are known e.g. from European Patent No. 199 262. This patent also discloses the use of the compounds of formula I for the treatment of convulsions of different origin, e.g. of epilepsy.

In accordance with the present invention, it has now surprisingly been found that the compounds of formula I are useful in the treatment of neuropathic pain.

The activity of the compounds of formula I in the treatment of neuropathic pain is evidenced, for example, in the following models of neuropathic pain in the rat and guineapig:

Wistar rats or Dunkin Hartley guinea pigs are anaesthetised with enflurane (in N₂O:O₂ for guinea pigs) and the left sciatic nerve is exposed and partially ligated with thread. This procedure produces a mechanical hyperalgesia which develops within 2-3 days and is maintained for at least 4 weeks. Paw withdrawal thresholds to a pressure stimulus are measured using an analgesymeter. Mechanical thresholds are taken on both the ipsilateral (ligated) and contralateral (unligated) paw prior to and then up to 6 hours following drug or vehicule administration. Reversal of hyperalgesia at each time point is calculated. Groups of 6 animals are used. Statistical analysis is carried out on withdrawal threshold readings using ANOVA followed by Tukey's HSD test.

In the rat model, the compounds of formula I significantly reverse neuropathic mechanical hyperalgesia at doses of about 10 to about 300 mg/kg p.o. With the compound 1-(2,6-difluorophenyl)methyl-1H-1,2,3-triazole-4-carboxamide, for example, a maximal reversal of neuropathic mechanical hyperalgesia of 30% is achieved after 3 hours on administration of 10 mg/kg p.o.

In the guinea pig model, the compounds of formula I significantly reverse neuropathic mechanical hyperalgesia at doses of about 3 to about 100 mg/kg p.o. With the above-mentioned carboxamide, for example, a maximal reversal of neuropathic mechanical hyperalgesia of 60% is achieved after 3 hours on administration of 30 mg/kg p.o.

The activity of the compounds of formula I in the treatment of neuropathic pain can be confirmed in clinical trials, for example in the following study aimed at evaluating the efficacy of a compound in treating chronic pain in patients with diabetic neuropathy:
Patients are randomized to receive 2400 mg/day of the compound or placebo in a 1:1 ratio.

The study consists of a Pre-randomization Phase (1 week) and a Double-blind Phase (5 weeks). The double-blind Phase consists of three periods: a one week Titration Period, a three-week Maintenance Period and a one-week Follow-up Period.

During the 1-week Pre-randomization Phase, the eligibility of the patients is evaluated. Patients meeting all inclusion/exclusion criteria are randomized to either the compound or placebo in the Double-blind Phase. During the 1-week Titration Period, study medication is up-titrated from 800mg/day (given b.i.d.) to 2400 mg/day (given b.i.d.). Patients who complete the 1-week Titration Period then enter the 3-week Maintenance Period. Patients who complete the 3-week Maintenance period or prematurely discontinue double-blind treatment then enter the 1-week Follow-up Period. Study medication is completely withdrawn on entry into the Follow-up Period. During the Double-blind Phase, serial efficacy and safety assessments are obtained.

120 male and female outpatients, aged 18-65 years with a clinical diagnosis of diabetes mellitus (type I or II) and a history of pain associated with diabetic neuropathy for 6 months to 3 years prior to study entry, are randomized 1:1 to the compound or placebo.

The total score of the Short-Form McGill Pain Questionnaire (SF-MPQ) at the end of Maintenence Period is used as primary efficacy parameter. Average weekly pain severity rating (daily patient pain diary) from start of randomized treatment to end of Maintenance Period, usage of rescue medication during the Titration and Maintenance Period, and average pain severity rating during the Follow-up Period (rebound pain), are used as secondary efficacy parameters.

The SF-MPQ total pain score at the end of the Maintenance Period is analyzed using an analysis of covariance model adjusting for the effect of treatment on post-treatment scores by using the baseline SF-MPQ total pain score as a covariate. Average weekly pain severity is analyzed using an analysis of covariance model with repeated measures using the treatment week and the mean pain severity rating during the Pre-randomization Phase as covariates. Usage of rescue medication during the Double-blind Phase is analyzed using the Cochran-Mantel-Haenszel test controlling for center. The mean pain severity rating during the Follow-up Period (rebound pain) is analyzed using an analysis of covariance model adjusting for the effect of treatment on the mean pain severity rating of the Follow-up Period with the mean pain severity rating during the Prerandomization Phase as a covariate.

In this study, the compounds of formula I, more particularly 1-(2,6-difluorophenyl)methyl-1H-1,2,3-triazole-4-carboxamide, are found to decrease pain severity ratings relative to placebo during the Maintenance and Follow-up Periods, in a statistically significant way.

The compounds of formula I are therefore useful in the treatment of neuropathic pain and associated hyperalgesia, including trigeminal and herpetic neuralgia, diabetic neuropathic pain, migraine, causalgia and deafferentation syndromes such as brachial plexus avulsion.

In a preferred group of formula I for use according to the invention, Ph is o-fluorophenyl, 2,5-difluorophenyl, 2,6-difluorophenyl or 2-chloro-6-fluorophenyl, R₁ is hydrogen or carbamoyl and R₂ is carbamoyl. The compound 1-(2,6-difluorophenyl)methyl-1H-1,2,3-triazole-4-carboxamide is particularly preferred.

For the above-mentioned indications the appropriate dosage will of course vary depending upon, for example, the compound employed, the host, the mode of administration and the nature and severity of the condition being treated. However, in general, satisfactory results in animals are indicated to be obtained at a daily dosage of from about 1 to about 50 mg/kg animal body weight. In larger mammals, for example humans, an indicated daily dosage is in the range from about 50 to about 3500 mg of a compound according to the invention conveniently administered, for example, in divided doses up to four times a day.

The compounds of formula I may be administered in any usual manner, e.g. orally, for example in the form of tablets or capsules, or parenterally, for example in the form of injection solutions or suspensions.

The present invention also provides the use of pharmaceutical compositions comprising a compound of formula I in association with at least one pharmaceutical carrier or diluent, in the treatment of neuropathic pain. Such compositions may be manufactured in conventional manner. Unit dosage forms may contain for example from about 10 mg to about 1500 mg of the compound of formula I.

For example tablets each containing 50 mg, or film-coated tablets each containing 100 mg, of 1-(2,6-difluorophenyl)methyl-1H-1,2,3-triazole-4-carboxamide, may be prepared as described in Examples 15 and 16 of EP 199262.

## Claims

1. Use of a compound of the formula 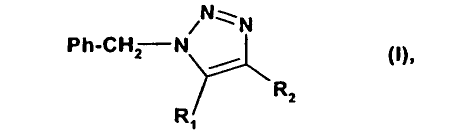 wherein Ph is o-fluorinated phenyl, which may be additionally substituted by 1 or 2 halogen atoms selected from the group consisting of fluorine and chlorine; R₁ is hydrogen, carbamoyl, N-(C₂-C₅)alkanoylcarbamoyl or N,N-di(C₁-C₄)alkylcarbamoyl; and R₂ is carbamoyl, N-(C₂-C₅)alkanoylcarbamoyl or N,N-di(C₁-C₄)alkylcarbamoyl, for the preparation of a pharmaceutical composition for the treatment of neuropathic pain.

2. Use according to claim 1, **characterized in that** the compound of the formula I is 1-(2,6-difluorophenyl)methyl-1H-1,2,3-triazole-4-carboxamide.

## Patentansprüche

1. Verwendung einer Verbindung der Formel 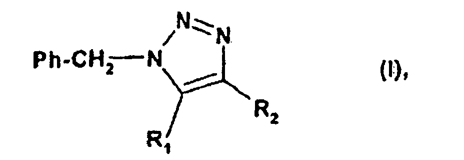 worin Ph für ein o-fluoriertes Phenyl steht, das zusätzlich substituiert sein kann durch 1 oder 2 Halogenatome, die ausgewählt sind aus der Gruppe, bestehend aus Fluor und Chlor; R₁ für Wasserstoff, Carbamoyl, N-(C₂-C₅)-Alkanoylcarbamoyl oder N,N-Di(C₁-C₄)-alkylcarbamoyl steht; und R₂ für Carbamoyl, N-(C₂-C₅)-Alkanoylcarbamoyl oder N,N-Di(C₁-C₄)-alkylcarbamoyl steht, für die Herstellung einer pharmazeutischen Zusammensetzung für die Behandlung von neuropathischem Schmerz.

2. Verwendung nach Anspruch 1, **dadurch gekennzeichnet, dass** die Verbindung der Formel I für 1-(2,6-Difluorphenyl)methyl-1H-1,2,3-triazol-4-carboxamid steht.

## Revendications

1. Utilisation d'un composé de formule 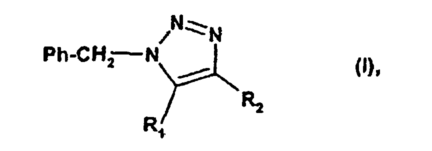 dans laquelle Ph est phényle o-fluoré, qui peut,de plus être substitué par 1 ou 2 atomes d'halogène sélectionnés à partir du groupe composé de fluor et de chlore ; R₁ est hydrogène, carbamoyle, N-(C₂-C₅)alkanoylcarbamoyle ou N,N-di(C₁-C₄)alkylcarbarmoyle ; et R₂ est carbamoyle, N-(C₂-C₅)alkanoyl carbamoyle ou N,N-di(C₁-C₄)alkylcarbamoyle, pour la préparation d'une composition pharmaceutique pour le traitement de la douleur neuropathique.

2. Utilisation selon la revendication 1, **caractérisée en ce que** le composé de formule I est le 1-(2,6-difluorophényl)méthyl-1H-1,2,3-triazole-4-carboxamide.
